(19) Europäisches Patentamt
European Patent Office
Office européen des brevets

(11) Veröffentlichungsnummer: **0 029 527**
**A2**

# (12) EUROPÄISCHE PATENTANMELDUNG

(21) Anmeldenummer: 80106759.6

(22) Anmeldetag: 04.11.80

(51) Int. Cl.³: C 07 C 177/00
A 61 K 31/557

(30) Priorität: 15.11.79 DE 2946116

(43) Veröffentlichungstag der Anmeldung: 03.06.81 Patentblatt 81/22

(84) Benannte Vertragsstaaten: AT BE CH DE FR GB IT LI NL

(71) Anmelder: HOECHST AKTIENGESELLSCHAFT
Postfach 80 03 20
D-6230 Frankfurt/Main 80(DE)

(72) Erfinder: Beck, Gerhard, Dr.
Gustav-Freytag-Strasse 24
D-6000 Frankfurt am Main(DE)

(72) Erfinder: Lerch, Ulrich, Dr.
Schwalbenweg 19
D-6238 Hofheim am Taunus(DE)

(72) Erfinder: Schölkens, Bernward, Dr.
Am Fliedergarten 1
D-6233 Kelkheim (Taunus)(DE)

(72) Erfinder: Rupp, Richard Helmut, Dr.
Johannesallee 24
D-6230 Frankfurt am Main 80(DE)

## (54) Prostaglandinderivate in der 6-Keto-PGA1-Reihe, Verfahren zu ihrer Herstellung sowie ihre Verwendung in Arzneimitteln und ihre Zwischenprodukte.

(57) Die vorliegende Erfindung betrifft Verbindungen der Formel I

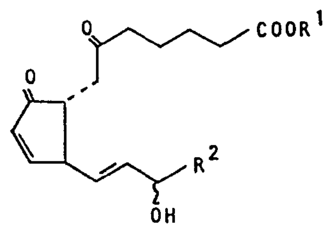

(1)

Sie zeichnen sich aus durch thrombocytenaggregationshemmende sowie blutdrucksenkende Wirkung und können daher als Arzneimittel verwendet werden.

EP 0 029 527 A2

BEZEICHNUNG GE...
siehe Titelseite



HOECHST AKTIENGESELLSCHAFT HOE 79/F 307 Dr. LA/Fr

Neue Prostaglandinderivate in der 6-Keto-$PGA_1$-Reihe, Verfahren zu ihrer Herstellung sowie ihre Verwendung als Arzneimittel.

Prostaglandine sind eine Gruppe von Fettsäuren, die in zahlreichen Geweben und Organen von Menschen und Tier vorkommen. Das Grundgerüst der natürlichen Prostaglandine besteht aus 20 Kohlenwasserstoffatomen, die in Form eines Fünfrings und zweier benachbarter linearer Seitenketten angeordnet sind.

Die pharmakologischen Effekte der Prostaglandine erstrecken sich u.a. auf die Gebiete der Reproduktion, des Bronchialmuskeltonus, des Blutdrucks und der Gastroenterologie. Die pharmakologischen Eigenschaften der natürlichen Prostaglandine sind Gegenstand zahlreiher Übersichtsartikel, z.B. N.H. Andersen und P.W. Ramwell in Arch. Internal Med. 133, 30 (1974); R.L. Jones in Pathobiology Ann. 1972, 359; J. Pike in Scient. American 225, 84 (1971) oder M.P.L. Caton in Progress in Med. Chem. vol. 7, ed.: Butterworth, London 1971.

Die Synthese von nicht natürlich vorkommenden Analogen von Prostansäuren, in denen die Vielzahl der pharmakologischen Wirkungen der natürlichen Prostaglandine differenziert sind, gewinnt zunehmend an Bedeutung.

Die vorliegende Erfindung betrifft neue Verbindungen der Formel I

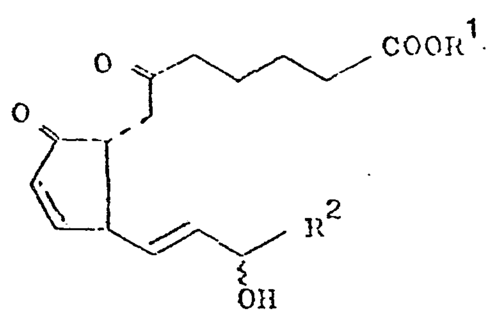

I

die strukturell mit den natürlichen Prostaglandinen verwandt sind und in welcher bedeuten

$R^1$ Wasserstoff oder einen geradkettigen oder verzweigten Alkylrest mit bis zu 8 Kohlenstoffatomen, einen geradkettigen oder verzweigten ungesättigten aliphatischen Kohlenwasserstoffrest mit bis zu 6 Kohlenstoffatomen, einen cycloaliphatischen Kohlenwasserstoffrest mit 3 bis 7 Kohlenstoffatomen, einen araliphatischen Kohlenwasserstoffrest mit 7 bis 9 Kohlenstoffatomen, oder ein physiologisch verträgliches Metall-, $NH_4$-oder Ammoniumion, das sich von einem primären, sekundären oder tertiären Amin ableitet, oder ein Tetraalkylammoniumion,

$R^2$ a) einen Cycloalkylrest mit 3 bis 7 Kohlenstoffatomen oder

b) einen geradkettigen oder verzweigten Alkylrest mit bis zu 8 Kohlenstoffatomen, in dem eine -$CH_2$-Gruppe durch ein Sauerstoffatom ersetzt sein kann, oder der substituiert sein kann

$b_1$) mit Halogen oder mit einem α- oder ß-Thienyl- oder Furylrest, die ihrerseits am Ring 1 - 3fach substituiert sein können mit Halogen, Trifluormethyl und/oder Alkyl oder Alkoxy mit je 1 - 6 C-Atomen oder

$b_2$) mit einem Phenoxyrest, einem α- oder ß-Thienyloxyrest, oder einem Cycloalkoxyrest mit 3 bis 7 Kohlenstoffatomen, wobei die genannten Reste ihrerseits am Ring 1 -3fach substituiert sein können mit Halogen, Trifluormethyl und/oder Alkyl oder Alkoxy mit je 1 - 6 C-Atomen.

Unter den für $R^1$ genannten Substituenten sind die

folgenden bevorzugt:
Wasserstoff, ein geradkettiger oder verzweigter Alkylrest mit bis zu 8 C-Atomen, ein geradkettiger oder verzweigter ungesättigter aliphatischer Kohlenwasserstoffrest mit bis zu 4 C-Atomen, ein cycloaliphatischer Kohlenwasserstoffrest mit 5 - 7 C-Atomen, oder ein araliphatischer Kohlenwasserstoffrest mit 7 bis 8 C-Atomen.

Insbesondere sind bevorzugt:
Wasserstoff, Methyl, Äthyl, n-Butyl, n-Pentyl, n-Hexyl, n-Heptyl, 2-Propyl, 2-Butyl, 2-Pentyl, 3-Hexyl, 2-Methylpropyl, 2-Methylbutyl, 4,4-Dimethylpentyl, 5,5-Dimethylhexyl, Cyclopentyl, Cyclohexyl, Cycloheptyl.

Unter den für $R^2$ genannten Substituenten sind die folgenden bevorzugt:
Cycloalkyl mit 5 bis 7 C-Atomen, geradkettige Alkylreste mit 3 bis 7 C-Atomen, verzweigte Alkylreste mit bis zu 8- C-Atomen, in denen eine nicht endständige $CH_2$-Gruppe durch ein Sauerstoffatom ersetzt sein kann, geradkettige oder verzweigte Alkylreste mit bis zu 5 C-Atomen, die substituiert sind mit Halogen, Thienyl, Furyl, Chlorthienyl, Phenoxy, Chlorphenoxy, Thienyloxy, Chlorthienyloxy oder Cyclohexyloxy.

Insbesondere sind bevorzugt:
1,1-Dimethyl-2-butoxy-äthyl, 1,1-Dimethyl-pentyl, n-Propyl, 2-Propyl, n-Butyl, t-Butyl, n-Pentyl, 3-Pentyl, 2-Methylbutyl, 2-Butyl, Neopentyl, n-Hexyl, 2-Äthylbutyl, 2,2-Dimethylbutyl, n-Heptyl, 1,1-Dimethyl-2-äthoxy-äthyl, 1,1-Dimethyl-2-methoxy-äthyl, 1,1-Dimethyl-cyclohexyloxymethyl, 1-Fluorpentyl, 1-Chlorpentyl, 5-Fluorpentyl, 5-Chlorpentyl, 3-Thienyl-2-äthyl, 2-Thienyl-2-äthyl, 3-(2-chlor-thienyl)-2-äthyl, 2(5-Chlor-thienyl)-2-äthyl, Phenoxymethyl, 3-Chlor-phenoxymethyl, 2-Thienyl-oxymethyl, 3(2-Chlorthienyl)-oxymethyl, 2(5-Chlorthienyl)-oxymethyl, 3-Furyl-2-äthyl, 2-Furyl-2-

äthyl, Cyclopentyl, Cyclohexyl, Cycloheptyl.

Gegenstand der Erfindung ist ferner ein Verfahren zur Herstellung von Verbindungen der Formel I, das dadurch gekennzeichnet ist, daß man

a) eine Verbindung der Formel II

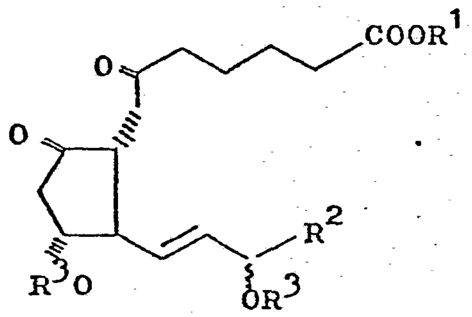

II

worin $R^1$ und $R^2$ die zur Formel I angegebene Bedeutung haben und $R^3$ Wasserstoff oder eine leicht abspaltbare Schutzgruppe bedeutet, in Gegenwart von starken Säuren in eine Verbindung der Formel I überführt,

b) gegebenenfalls eine Verbindung der Formel I, in der $R^1$ Wasserstoff bedeutet, verestert zu einer Verbindung der Formel I, worin $R^1$ die zur Formel I gegebene Bedeutung außer Wasserstoff besitzt,

c) gegebenenfalls eine Verbindung der Formel I, in der $R^1$ nicht Wasserstoff oder ein Kation darstellt, durch saure oder alkalische Hydrolyse überführt in eine Verbindung der Formel I, in der $R^1$ Wasserstoff oder ein physiologisch verträgliches Kation bedeutet, und

d) gegebenenfalls in einer Verbindung der Formel I, in der $R^1$ ein physiologisch verträgliches Metall-, $NH_4$- oder Ammoniumion, das sich von einem primären, sekundären oder tertiären Amin ableitet oder ein Tetraalkylammoniumion bedeutet, das Kation $R^1$ gegen ein anderes austauscht.

Die Herstellung der im erfindungsgemäßen Verfahren als Ausgangsmaterial verwendeten Prostaglandinderivate der Formel II bzw. der Formel III erfolgt wie in der DE-OS 29 13 856 (HOE 79/F 086) beschrieben.

Die Verbindungen der Formel II, worin $R^3$ eine Schutzgruppe darstellt, können z.B. durch Behandlung mit 2%iger wäßrig-alkoholischer Oxalsäurelösung bei 20 bis 50°C in Verbindungen der Formel II, worin $R^3$ Wasserstoff bedeutet, umgewandelt werden.

Die Verbindungen der Formel II werden mit einer starken Säure, wie z.B. wässriger Salzsäure bei pH 0,9 - 2,0 und bei Temperaturen von 0 bis +50°C, vorzugsweise bei +25 bis +35°C in Verbindungen der Formel I überführt.

Die Veresterung von Verbindungen der Formel I ($R^1$ = H) erfolgt nach üblichen Methoden, z.B. wie sie in der DE-OS 26 28 564 (HOE 76/F 149) beschrieben sind.

Verbindungen der Formel I, worin $R^1$ einen Alkylrest bedeutet, können in üblicher Weise in alkalischem Medium zu Verbindungen der Formel I, worin $R^1$ Wasserstoff oder vorzugsweise ein Kation bedeutet, verseift werden, z.B. mit NaOH oder KOH in einem niedermolekularen Alkohol wie Methanol oder Äther wie Dimethoxyäthan oder THF, gegebenenfalls in Gegenwart von Wasser. Vorteilhafterweise benutzt man die äquimolare Menge oder einen sehr geringen Überschuß an Alkalihydroxid, so daß man das Alkalisalz der Formel I ($R^1$ = Alkalimetallion) durch Verdampfen des Lösungsmittels erhält. Das Alkalikation läßt sich an Ionenaustauschern in üblicher Weise gegen beliebige Kationen austauschen. Dazu läßt man die Lösung des Alkalisalzes einer Verbindung der Formel I durch eine mit einem Kationaustauscher wie z.B. (R)Amberlite CG - 50 oder (R)Dowex CCR - 2 gefüllte Säule laufen.

Der Kationenaustauscher ist mit dem erwünschten Kation beladen, z.B. mit einem Ammoniumion, das sich von einem primären, sekundären oder tertiären Amin ableitet. Das erwünschte Salz erhält man durch Eindampfen des Eluats. Die Verbindungen der Formel I und II können als Diastereomerengemisch bezüglich der Stellung der Hydroxylgruppe am Kohlenstoffatom 15 (Prostaglandinnomenklatur), als reine α- oder ß-Isomeren oder in Form von optisch aktiven Antipoden für die nachfolgenden Reaktionen eingesetzt werden. Die Trennung von Stereoisomeren bzw. Antipoden kann aber auch nach jeder der folgenden Reaktionsstufe erfolgen. Das bedeutet, daß alle beschriebenen R-aktionen mit Diastereomerengemischen, reinen Diastereomeren oder optisch aktiven Antipoden durchgeführt werden können.

Sofern die einzelnen Reaktionsprodukte nicht bereits in genügend reiner Form anfallen, so daß sie für den folgenden Reaktionsschritt eingesetzt werden können, empfiehlt sich eine Reinigung mittels z.B. Säulen-, Dünnschicht- oder Hochdruckflüssigkeitschromatographie.

Nach den erfindungsgemäßen Verfahren lassen sich außer den in den Beispielen genannten insbesondere auch die folgenden Verbindungen herstellen:

16-Chlor-6-Keto-$PGA_1$-methylester
16-Fluor-6-Keto-$PGA_1$
16-Chlor-6-Keto-$PGA_1$-äthylester
17-(2-Thienyl)-18,19,20-trinor-6-Keto-$PGA_1$
17-(3-(2-Chlorthienyl)-18,19,20-trinor-6-Keto-$PGA_1$-methylester
16-Phenoxy-17,18,19,20-tetranor-6-Keto-$PGA_1$-äthylester
16-(3-Trifluormethyl-phenoxy)-17,18,19,20-tetranor-6-Keto-$PGA_1$-n-butylester
16-(3-(2-Chlor)-thienyloxy)-17,18,19,20-tetranor-6-Keto-$PGA_1$-methylester

16-(2-Thienyloxy)-17,18,19,20-tetranor-6-Keto-$PGA_1$-methylester
17-(3-Furyl)-18,19,20-trinor-6-Keto-$PGA_1$
15-Cyclopentyl-16,17,18,19,20-pentanor-6-Keto-$PGA_1$-propylester
15-Cyclopentyl-16,17,18,19,20-pentanor-6-Keto-$PGA_1$
17-Oxa-6-Keto-$PGA_1$-methylester
16,16-Dimethyl-17-oxa-21-homo-6-Keto-$PGA_1$
17-Oxa-16,16,19-trimethyl-6-Keto-$PGA_1$-n-butylester
16,16-Dimethyl-18-oxa-21-homo-6-Keto-$PGA_1$
19-Oxa-6-Keto-$PGA_1$-äthylester
16,16-Diäthyl-19-oxa-6-Keto-$PGA_1$-methylester
19-Oxa-16,16,20,20-tetramethyl-6-Keto-$PGA_1$-isopropylester
18,18-Dimethyl-19-oxa-6-Keto-$PGA_1$
16,16-Dimethyl-20-oxa-21-homo-6-Keto-$PGA_1$-methylester

Die Verbindungen der Formel I zeichnen sich aus durch hemmende Wirkung auf die Thrombocytenaggregation sowie blutdrucksenkende Eigenschaften.

Sie können daher als Arzneimittel angewandt werden. Die Anwendung der Verbindungen der Formel I als Blutdrucksenker erfolgt im Tagesdosisbereich von 0,001 mg - 0,1 mg/kg, vorzugsweise 0,005 mg - 0,1 mg/kg bei i.v. Applikation bzw. Tagesdosisbereich von 0,01 mg - 1 mg/kg, vorzugsweise 0,05 - 1 mg/kg bei oraler Applikation. Die Einheitsdosis beträgt 1/3 der angegebenen Tagesdosen. Zur Hemmung der Thrombocytenaggregation sind teilweise auch niedrigere Dosierungen wie oben angegeben wirksam.

Die erfindungsgemäßen Verbindungen der Formel I können als freie Säuren, oder in Form ihrer physiologisch unbedenklichen anorganischen oder organischen Salze oder als Ester zur Anwendung kommen. Säuren und Salze bzw. Ester können in Form ihrer wässrigen Lösungen oder Suspensionen oder auch gelöst oder suspendiert in

pharmakologisch unbedenklichen organischen Lösungsmitteln wie ein- oder mehrwertigen Alkoholen wie z.B. Äthanol, Äthylenglykol oder Glycerin, Ölen wie z.B. Sonnenblumenöl oder Lebertran, Äthern wie z.B. Diäthylenglykoldimethyläther oder auch Polyäthern wie z.B. Polyäthylglykol oder auch in Gegenwart anderer pharmakologisch unbedenklicher Polymerträger wie z.B. Polyvinylpyrrolidon zur Anwendung gelangen. Als Zubereitungen können die üblichen galenischen Infusions- oder Injektionslösungen und Tabletten, sowie örtlich anwendbare Zubereitungen wie Cremes, Emulsionen, Suppositorien oder Aerosole in Frage kommen.

Eine weitere Anwendung der neuen Verbindungen liegt in der Kombination mit anderen Wirkstoffen. Neben anderen geeigneten Substanzen gehören dazu vor allem:

Kreislaufmittel im weitesten Sinne, wie z.B. Herzglycoside wie Digitoxin, Sympathomimetica wie Suprifen, ß-Sympatholytica wie Inderal, Coronardilatatoren wie Chromonar oder Prenylamin, blutdrucksenkende Stoffe wie Reserpin oder Clonidin, Antiarrhythmica, durchblutungsfördernde Stoffe, Antikoagulantien oder Fibrinolytika, Diuretica wie z.B. Furosemid, Lipidsenker, Prostaglandine oder Prostaglandinantagonisten oder Prostaglandin-Biosynthesehemmer, wie z.B. nichtsteroidale Antiphlogistika, Thromboxan-Synthetasehemmer, Psychopharmaka sowie Vitamine.

Die Verbindungen der Formel II sind wertvolle Zwischenprodukte für die Herstellung von Verbindungen der Formel I.

Beispiel 1:

6-Keto-16(3-thienyloxy)-17,18,19,20-tetranor-$PGA_1$ (I)
(15α-Epimeres)

100 mg (0,175 mMol) 6-Keto-11,15-bis-tetrahydropyranyloxy-16(3-thienyloxy)-17,18,19,20-tetranor-$PGE_1$-methylester (III) (hergestellt wie in DE-OS 29 13 856 beschrieben) werden in 2 ml Tetrahydrofuran unter Stickstoff gelöst und tropfenweise unter Rühren 1 ml 2 N Salzsäure zugegeben. Dann wird ca. 5 Std. bei 45°C gerührt. Der Reaktionsverlauf wird im Dünnschichtchromatogramm verfolgt. (Essigester/Cyclohexan/Eisessig 80/20/1). Nach beendeter Reaktion werden 10 ml Wasser zugegeben und 4 mal mit 30 ml Essigester die wässrige Phase extrahiert. Die vereinigten organischen Phasen werden mit $MgSO_4$ getrocknet, filtriert und das Lösungsmittel im Vakuum entfernt.

Rohausbeute: 98 mg gelbes Öl

Auf einer Lobar ®- Kieselgelfertigsäule Größe A (240 - 10) Li Chroprep ® Si 60 (40 - 63 μm) der Fa. Merck Darmstadt wird das Rohprodukt chromatographiert (Essigester/Cyclohexan/Eisessig 60/40/1)

Reinausbeute: Fraktion: 14 - 19 = 23,4 mg (34,1% d.Th.)

Dünnschichtchromatogramm:

$R_f$ Wert: 0,41 (Essigester/Cyclohexan/Eisessig = 80/20/1)

(UV-detektion oder Platte Anfärben mit Molybdatophosphorsäure in Methanol, Platte auf 100°C erwärmen)

0029527

Beispiel 1 b bis 1 k:

In Analogie zu Beispiel 1 a lassen sich die Verbindungen der Beispiele 1 b bis 1 k durch Abspaltung der Schutzgruppen und 1 Mol Wasser herstellen.

| Beispiel 1 | $R^2$ | $R_f$ Werte | δ-Werte NMR ($CDCl_3$) |
|---|---|---|---|
| a) | 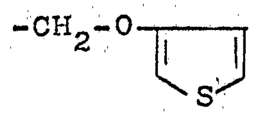<br>Rein-Ausbeute %<br>34,1 | 0,41 | 1,5-1,9 (m,4H) $-CH_2-$;<br>2,1-2,6 (m,6H) $-CH_2CO-$;<br>2,65-2,9 (m,1H) -CH-CO;<br>3,1-3,5 (m,1H) -<u>CH</u>-CH=CH-;<br>3,7-3,9 (d,2H) $-CH_2O-$;<br>4,0-4,1 (m,1H) <u>CH</u>-OH;<br>4,8-5,6 (breites s, 2H) OH,COOH;<br>5,6-5,9 (m,2H) CH=CH;<br>6,1-7,2 (3xm, 3H + 1H)<br>Thiophen + CH=<u>CH</u>-CO-;<br>7,4-7,6 (m,1H) <u>CH</u>=CH-CO; |

| Beispiel 1 | $R^2$ | $R_f$ Werte | δ-Werte NMR ($CDCl_3$) |
| --- | --- | --- | --- |
| b) | $-CH_2-CH_2-$ 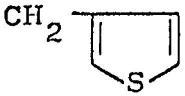<br><br>Rein-Ausbeute %<br>46 | 0,39 | 1,0-2,0 (m,8H) $-CH_2-$;<br>2,1-2,6 (m,6H) $-CH_2CO$;<br>2,65-2,9 (m,1H) -CH-CO;<br>3,15-3,45 (m,1H) -CH-CH=CH-;<br>4,0-4,1 (m,1H) CH-OH;<br>4,3-5,2 (breites s, 2H) OH, COOH;<br>5,6-5,8 (m,2H) CH=CH;<br>6,1-6,3 (m,1H) CH=CH-CO;<br>6,85-7,0 (m,2H) Thiophen;<br>7,1-7,35 (m,1H) Thiophen;<br>7,4-7,6 (m,1H) <u>CH</u>=CH-CO; |

| Beispiel 1 | $R^2$ | $R_f$ Werte | $\delta$-Werte NMR ($CDCl_3$) |
|---|---|---|---|
| c) | 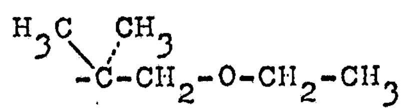<br><br>Rein-Ausbeute %<br><br>38,5 | 0,46 | 0,9 (s,6H) $C(CH_3)_2$;<br>1,15 (t,3H) $CH_2\underline{CH_3}$;<br>1,45-1,9 (m,4H) $-CH_2-$;<br>2,2-2,6 (m,6H) $-CH_2-CO-$;<br>2,6-2,9 (m,1H) -CH-CO;<br>3,1-3,6 (s,q u. m,5H) $-CH_2-O-$,<br>$-\underline{CH}-CH=CH-$;<br>3,9-4,05 (m,1H) $\underline{CH}-OH$;<br>4,2-5,0 (breites s,2H) OH, COOH;<br>5,55-5,7 (m,2H) CH=CH;<br>6,1-6,3 (m,1H) $CH=\underline{CH}-CO$;<br>7,4-7,6 (m,1H) $C\underline{H}=CH-CO$; |

| Beispiel 1 | $R^2$ | $R_f$ Werte | $\delta$-Werte NMR ($CDCl_3$) |
|---|---|---|---|
| d) | 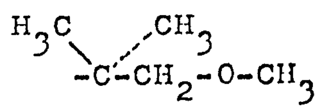<br>Rein-Ausbeute %<br>49,7 | 0,45 | 0,9 (s,6H) $C(CH_3)_2$;<br>1,4-1,9 (m,4H) $-CH_2-$;<br>2,2-2,6 (m,6H) $-CH_2-CO-$;<br>2,6-2,9 (m,1H) $-CH-CO$;<br>3,2 (s,2H) $-\underline{CH}_2-OCH_3$;<br>3,3 (s,3H) $-CH_2-O\underline{CH}_3$;<br>3,15-3,5 (m,1H) $-C\underline{H}-CH=CH-$;<br>3,9-4.05 (m,1H) $C\underline{H}-OH$;<br>4,3-4,9 (breites s,2H) OH, COOH;<br>5,5-5,8 (m,2H) $CH=CH$;<br>6,1-6,3 (m,1H) $CH=\underline{CH}-CO$;<br>7,4-7,6 (m,1H) $\underline{CH}=CH-CO$; |

| Beispiel 1 | $R^2$ | $R_f$ Werte | δ-Werte NMR ($CDCl_3$) |
|---|---|---|---|
| e) | 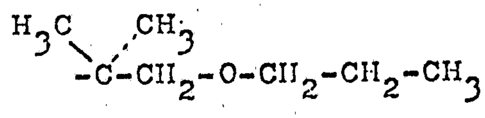<br><br>Rein-Ausbeute %<br><br>35 | 0,53 | 0,9 (s + t,9H) $-C(CH_3)_2$, $CH_3$;<br>1,5-1,9 (m,6H) $-CH_2-$;<br>2,1-2,7 (m,6H) $-CH_2-CO-$;<br>2,7-2,9 (m,1H) $-CH-CO$;<br>3,1-3,6 (s,q u. m, 5H) $-CH_2O-$,<br>$-\underline{CH}-CH{=}CH-$;<br>3,9-4,02 (m,1H) $\underline{CH}-OH$,<br>4,8-5,6 (breites s, 2H) OH, COOH;<br>5,55-5,7 (m,2H) $CH{=}CH$;<br>6,1-6,3 (m,1H) $CH{=}\underline{CH}-CO$;<br>7,4-7,6 (m,1H) $\underline{CH}{=}CH-CO$; |

| Beispiel 1 | $R^2$ | $R_f$ Werte | δ-Werte NMR ($CDCl_3$) |
| --- | --- | --- | --- |
| f) | 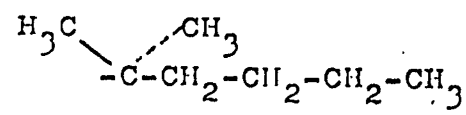<br>Rein-Ausbeute %<br>30 | 0,51 | 0,9 (s + t,9H) $-C(CH_3)_2$, $CH_3$;<br>1,0-1,4 (m,6H) $-CH_2-$;<br>1,45-1,95 (m,4H) $-CH_2-$;<br>2,1-2,6 (m,6H) $-CH_2-CO-$;<br>2,5-2,95 (m,1H) -<u>CH</u>-CO;<br>3,1-3,5 (m,1H) -<u>CH</u>-CH=CH-;<br>3,9-4,0 (m,1H) <u>CH</u>-OH;<br>4,2-4,9 (breites s,2H) OH, COOH;<br>5,5-5,7 (m,2H) -CH=CH-;<br>6,1-6,3 (m,1H) CH=<u>CH</u>-CO;<br>7,4-7,6 (m,1H) <u>CH</u>=CH-CO; |

| Beispiel 1 | $R^2$ | $R_f$ Werte | δ-Werte NMR ($CDCl_3$) |
|---|---|---|---|
| g) | $\overset{F}{\overset{\vert}{-CH}}-CH_2-CH_2-CH_2-CH_3$<br><br>Rein-Ausbeute %<br>48 | 0,43 | 0,95 (t,3H) $CH_3$;<br>1,0-1,9 (m,10H) $-CH_2-$;<br>2,1-2,6 (m,6H) $-CH_2-CO-$;<br>2,6-2,9 (m,1H) -<u>CH</u>-CO;<br>3,1-3,5 (m,1H) -<u>CH</u>-CH=CH-;<br>3,9-4,0 (m,1H) -<u>CH</u>OH;<br>4,3-5,0 (breites s,2H + 1H) OH, COOH, -<u>CH</u>-F;<br>5,55-5,75 (m,2H) -CH=CH-;<br>6,1-6,3 (m,1H) CH=<u>CH</u>-CO;<br>7,4-7,6 (m,1H) <u>CH</u>=CH-CO; |

| Beispiel 1 | $R^2$ | $R_f$ Werte | δ -Werte NMR ($CDCl_3$) |
|---|---|---|---|
| h) | 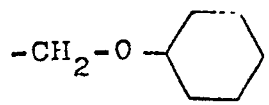<br>Rein-Ausbeute %<br>51 | 0,40 | 3,75-3,85 (d,2H) $\underline{CH_2}$-O-$C_6H_5$;<br>6,7-7,4 (m,5H) aromat. Protonen.<br>olefinische Protonen entsprechend Beispiel a) |
| i) | 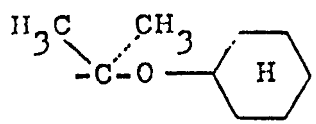<br>Rein-Ausbeute %<br>38 | 0,5 | 1,05 (d,6H) $C(CH_3)_2$;<br>0,9-4,0 (m,23H) $-CH_2-$, $CH_2-CO$,<br>-CH-CO, -CH-CH=CH-, C$\underline{H}$-OH;<br>4,4-5,2 (breites s, 2H) OH, COOH<br>olefinische Protonen entsprechend Beispiel k) |

| Beispiel 1 | $R^2$ | $R_f$ Werte | $\delta$-Werte NMR ($CDCl_3$) |
|---|---|---|---|
| j) | 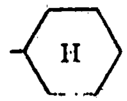<br>Rein-Ausbeute %<br>33 | 0,54 | 0,9-4,1 (m,23H) $-CH_2-$, $CH_2-CO$, $-CH-CO$, $-\underline{CH}-CH=CH-$, $CH-OH$;<br>4,6-5,4 (breites s,2H) OH, COOH<br>olefinische Protonen entsprechend Beispiel k) |
| k) | $-C_5H_{11}$<br>Rein-Ausbeute %<br>25 | 0,4 | 0,9 (t,3H) $CH_3$;<br>1,05-1,85 (m,12H) $-CH_2-$;<br>2,1-2,6 (m,6H) $-CH_2-CO-$;<br>2,6-2,95 (m,1H) $-\underline{CH}-CO$;<br>3,1-3,45 (m,1H) $-\underline{CH}-CH=CH-$;<br>3,9-4,1 (m,1H) $\underline{CH}-OH$;<br>4,0-4,9 (breites s,2H) OH, COOH;<br>5,5-5,7 (m,2H) $-CH=CH-$;<br>6,1-6,3 (m,1H) $CH=\underline{CH}-CO$;<br>7,4-7,6 (m,1H) $\underline{CH}=CH-CO$; |

Beispiel 2 a:

6-Keto-16(3-thienyloxy)-17,18,19,20-tetranor-$PGA_1$ I (15α-Epimeres)

151 mg (0,385 mMol) 6-Keto-16(3-thienyloxy)-17,18,19,20-tetranor-$PGE_1$-methylester (II) (hergestellt wie in HOE 79 F 086 beschrieben) werden in 5 ml Tetrahydrofuran unter Stickstoff gelöst und tropfenweise unter Rühren 1 ml 1,5 N Salzsäure zugegeben. Dann wird ca. 3 Std. bei 40°C gerührt. Der Reaktionsverlauf wird im Dünnschichtchromatogramm verfolgt. (Essigester/Cyclohexan/Eisessig 80/20/1). Nach beendeter Reaktion werden 10 ml Wasser zugegeben und 4 mal mit 30 ml Essigester die wässrige Phase extrahiert. Die vereinigten organischen Phasen werden mit $MgSO_4$ getrocknet, filtriert und das Lösungsmittel im Vakuum entfernt.

Rohausbeute: 143 mg gelbes Öl

Auf einer Lobar $^R$- Kieselgelfertigsäule Größe A (240 - 10) Li Chroprep $^R$ Si 60 (40 - 63 m) der Fa. Merck Darmstadt wird das Rohprodukt chromatographiert (Essigester/Cyclohexan/Eisessig 60/40/1)

Reinausbeute: Fraktion: 11 - 32 = 81.2 mg (54 % d. Th.)

Dünnschichtchromatogramm:

$R_f$ Wert: 0,41 (Essigester/Cyclohexan/Eisessig = 80/20/1)

(UV-detektion oder Platte Anfärben mit Molybdatophosphorsäure in Methanol, Platte auf 100°C erwärmen)

Beispiel 2 b bis 2 K:

In Analogie zu Beispiel 2 a lassen sich die Verbindungen der Beispiele 2 b bis 2 K durch Abspaltung von 1 Mol Wasser herstellen (Formel I) $R^1 = H$

Die analytischen Daten der so hergestellten Verbindungen der allg. Formel I ($R_1$=H) wie $R_f$-wert und NMR spektren entsprechen den unter Beispiel 1 a - 1 K angegebenen Daten.

Beispiel 3 a:

6-Keto-16,16-Dimethyl-18-oxa-$PGA_1$-methylester (I)
(15α-Epimeres)

20,1 mg (0,0528 m Mol) 6-Keto-16,16-Dimethyl-18-oxa-$PGA_1$ (Beispiel 1 c oder 2 c) werden in 3 ml Diäthyläther gelöst und mit 0,95 ml einer 0,1 molaren Diazomethanlösung in Diäthyläther versetzt. Nach kurzem Rühren wird das Lösungsmittel im Vakuum entfernt. Aus dem Dünnschichtchromatogramm ergibt sich der Reaktionsverlauf.
Ausbeute: 20.5 mg helles Öl (98 % d. Th.)

NMR -werte: analog Beispiel 1 c bzw. 2 c
in $CDCl_3$ Estersignal $\delta = 3.6$ (s, 3H)

$R_{f\ Wert}$: 0.56 (Cyclohexan/Essigester/Eisessig = 40/60/1)

(Ausgangsmaterial $R_{f\ Wert}$: 0.27)

Beispiele 3 b - 3 w:

In Analogie zu Beispiel 3 a lassen sich aus den Verbindungen der Beispiele 1 a bis 1 h bzw. 2 a - 2 h durch Umsetzung mit Diazomethan die Methylester 3 a bis 3 h herstellen (Formel I) $R^1 = CH_3$. Andere Ester lassen sich entsprechend durch Ersatz von Diazomethan durch Diazoalkan herstellen. Andere Methoden der Veresterung erfolgen wie in der DE-OS 26 28 564 (HOE 76 F 149) beschrieben.

Beispiel 4 a:

Natriumsalz des 6-Keto-16(13-thienylody)-17,18,19,20-tetranor-$PGA_1$ I

196,3 mg (0,5 mMol) 6-Keto-16(3-thienyloxy)-17-18-19-20-tetranor $PGA_1$ (Beispiel 1 a) werden in 20 ml Äthanol gelöst. Zu dieser Lösung gibt man unter Rühren bei -20°C eine Lösung von 12 mg Natrium in 4 ml Äthanol. Man rührt 1/4 Std. unter Argon., und entfernt das Lösungsmittel im Vakuum bei - 15°C (Gefriertrocknung). Man erhält das Natriumsalz des Prostaglandinderivates als farbloses Pulver.

In Analogie zu Beispiel 4 a lassen sich aus den Verbindungen der Beispiele 1 b - 1 k bzw. 2 b - 2 k die Natriumsalze herstellen (Formel I) $R^1$ = Na

BAD ORIGINAL

Beispiel 5 a:

Trishydroxymethylaminomethansalz des 6-Keto-16(3-thienyloxy)-17,18,19,20-tetranor-$PGA_1$ I

Eine wäßrige alkoholische Lösung von 98 mg 6-Keto-16(3-thienyloxy)-17,18,19,20-tetranor-$PGA_1$ (Beispiel 1 a) in 50 ml Wasser-Äthanol (1:1) wird bei $-15^{o}C$ mit einer Lösung von 0,25 mMol Trishydroxymethylaminomethan-base in 10 ml Wasser versetzt. Die wässrig alkoholische Lösung wird sofort gefriergetrocknet bei $-15^{o}C$. Man erhält das Trisalz des Prostaglandinderivates als Krist. weißer Pulver. (Zersp. > $40^{o}C$)

In Analogie zu Beispiel 5 a lassen sich aus den Verbindungen der Beispiele 1 b - 1 k bzw. 2 b - 2 k die Trisalze herstellen (Formel I) $R^1$ =

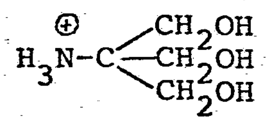

Patentansprüche:

1. Verbindungen der Formel I

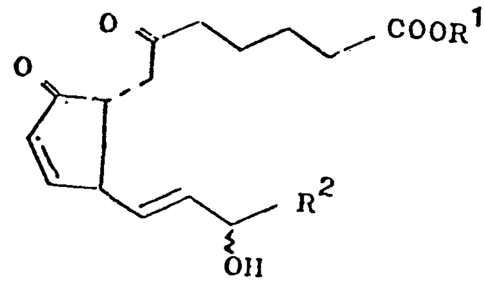

I

in welcher bedeuten:

$R^1$ Wasserstoff oder einen geradkettigen oder verzweigten Alkylrest mit bis zu 8 Kohlenstoffatomen oder einen geradkettigen oder verzweigten ungesättigten aliphatischen Kohlenwasserstoffrest mit bis zu 6 Kohlenstoffatomen oder einen cycloaliphatischen Kohlenwasserstoffrest mit 3 bis 7 Kohlenstoffatomen oder einen araliphatischen Kohlenwasserstoffrest mit 7 bis 9 Kohlenstoffatomen oder ein physiologisch verträgliches Metall-, $NH_4$- oder Ammoniumion, das sich von einem primären, sekundären oder tertiären Amin ableitet, oder ein Tetraalkylammoniumion,

$R^2$ a) einen Cycloalkylrest mit 3 bis 7 Kohlenstoffatomen oder

b) einen geradkettigen oder verzweigten Alkylrest mit bis zu 8 Kohlenstoffatomen, in dem eine $-CH_2$-Gruppe durch ein Sauerstoffatom ersetzt sein kann, oder der substituiert sein kann

$b_1$) mit Halogen oder mit einem α- oder ß-Thienylrest oder einem α- oder ß-Furylrest, die ihrerseits am Ring 1 - 3fach substituiert sein können mit Halogen, Trifluormethyl und/oder Alkyl oder Alkoxy mit je 1 bis 6 C-Atomen oder

$b_2$) mit Phenyloxy-, einem α- oder ß-Thienyloxy- oder einem Cycloalkoxyrest mit 3 bis 7 Kohlenstoffatomen, wobei die genannten Reste ihrerseits am Ring 1 - 3fach substituiert sein können mit

Halogen, Trifluormethyl und/oder Alkyl oder Alkoxy mit je 1 - 6 C-Atomen.

2. Verbindungen der Formel II

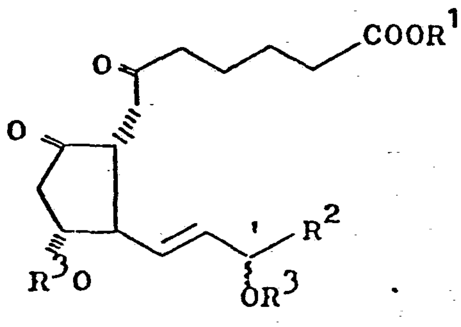

II

worin $R^1$ und $R^2$ die zur Formel I angegebene Bedeutung haben und $R^3$ Wasserstoff oder eine leicht abspaltbare Schutzgruppe bedeutet.

3. Verfahren zur Herstellung von Verbindungen der Formel I, dadurch gekennzeichnet, daß man

a) eine Verbindung der Formel II

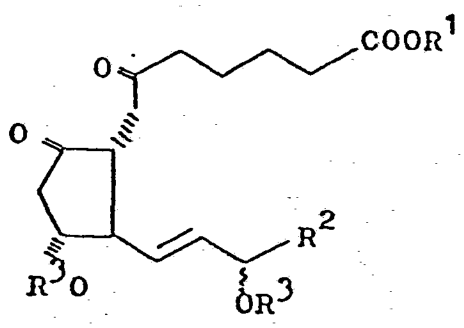

II

worin $R^1$ und $R^2$ die zur Formel I angegebene Bedeutung haben und $R^3$ Wasserstoff oder eine leicht abspaltbare Schutzgruppe bedeutet, in Gegenwart von starken Säuren in eine Verbindung der Formel I überführt,

b) gegebenenfalls eine Verbindung der Formel I, in der $R^1$ Wasserstoff bedeutet, verestert zu einer Verbindung der Formel I, worin $R^1$ die zur Formel I angegebene Bedeutung außer Wasserstoff besitzt,

c) gegebenenfalls eine Verbindung der Formel I, in der

$R^1$ nicht Wasserstoff oder ein Kation darstellt, durch Hydrolyse überführt in eine Verbindung der Formel I, in der $R^1$ Wasserstoff oder ein physiologisch verträgliches Kation bedeutet und

d) gegebenenfalls in einer Verbindung der Formel I, in der $R^1$ ein physiologisch verträgliches Metall-, $NH_4$-oder Ammoniumion, das sich von einem primären, sekundären oder tertiären Amin ableitet oder ein Tetraalkylammoniumion bedeutet, das Kation $R^1$ gegen ein anderes austauscht.

4. Verfahren zur Herstellung von pharmazeutischen Präparaten zur Behandlung von Thrombosen und/oder Infarkten sowie zur Behandlung des Bluthochdrucks, dadurch gekennzeichnet, daß man eine Verbindung der Formel I in Anspruch 1, gegebenenfalls mit pharmazeutisch üblichen Trägerstoffen und/oder Stabilisatoren in eine therapeutisch geeignete Anwendungsform bringt.

5. Pharmazeutische Präparate zur Behandlung von Thrombosen und/oder Infarkten sowie zur Behandlung des Bluthochdrucks, gekennzeichnet durch einen Gehalt an einer Verbindung der Formel I in Anspruch 1.

6. Verwendung einer Verbindung der Formel I bei der Bekämpfung von Thrombosen und/oder Infarkten sowie bei der Behandlung des Bluthochdrucks.

Patentanspruch für Österreich:

Verfahren zur Herstellung von Verbindungen der Formel I

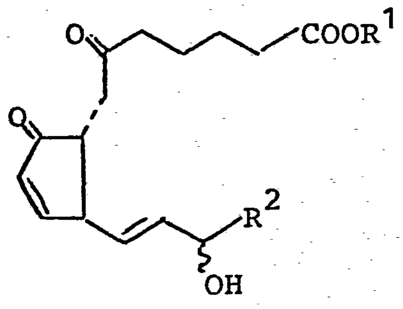

(I)

die strukturell mit den natürlichen Prostaglandinen verwandt sind und in welcher bedeuten

$R^1$ Wasserstoff oder einen geradkettigen oder verzweigten Alkylrest mit bis zu 8 Kohlenstoffatomen, einen geradkettigen oder verzweigten ungesättigten aliphatischen Kohlenwasserstoffrest mit bis zu 6 Kohlenstoffatomen, einen cycloaliphatischen Kohlenwasserstoffrest mit 3 bis 7 Kohlenstoffatomen, einen araliphatischen Kohlenwasserstoffrest mit 7 bis 9 Kohlenstoffatomen, oder ein physiologisch verträgliches Metall-, $NH_4$- oder Ammoniumion, das sich von einem primären, sekundären oder tertiären Amin ableitet, oder ein Tetraalkylammonium,

$R^2$ a) einen Cycloalkylrest mit 3 bis 7 Kohlenstoffatomen oder

b) einen geradkettigen oder verzeigten Alkylrest mit bis zu 8 Kohlenstoffatomen, in dem eine $-CH_2$-Gruppe durch ein Sauerstoffatom ersetzt sein kann, oder der substituiert sein kann

$b_1$) mit Halogen oder mit einem α- oder ß-Thienyl- oder Furylrest, die ihrerseits am Ring 1 - 3fach substituiert sein können mit Halogen, Trifluormethyl und/oder Alkyl oder Alkoxy mit je 1 - 6 C-Atomen oder

$b_2$) mit einem Phenoxyrest, einem α- oder ß-Thienyloxyrest, oder einem Cycloalkoxyrest mit 3 bis 7 Kohlenstoffatomen, wobei die genannten Reste ihrerseits am Ring

1 - 3fach substituiert sein können mit Halogen, Trifluormethyl und/oder Alkyl oder Alkoxy mit je 1 - 6 C-Atomen,

dadurch gekennzeichnet, daß man

a) eine Verbindung der Formel II

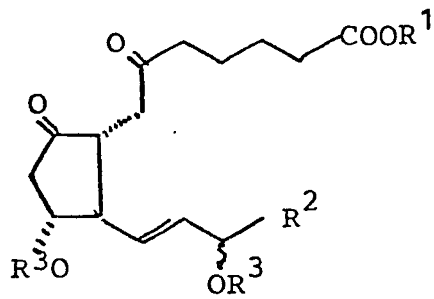

(II)

worin $R^1$ und $R^2$ die zur Formel I angegebene Bedeutung haben und $R^3$ Wasserstoff oder eine leicht abspaltbare Schutzgruppe bedeutet, in Gegenwart von starken Säuren in eine Verbindung der Formel I überführt,

b) gegebenenfalls eine Verbindung der Formel I, in der $R^1$ Wasserstoff bedeutet, verestert zu einer Verbindung der Formel I, worin $R^1$ die zur Formel I angegebene Bedeutung außer Wasserstoff besitzt,

c) gegebenenfalls eine Verbindung der Formel I, in der $R^1$ nicht Wasserstoff oder ein Kation darstellt, durch Hydrolyse überführt in eine Verbindung der Formel I, in der $R^1$ Wasserstoff oder ein physiologisch verträgliches Kation bedeutet und

d) gegebenenfalls in einer Verbindung der Formel I, in der $R^1$ ein physiologisch verträgliches Metall-, $NH_4$- oder Ammoniumion, das sich von einem primären, sekundären oder tertiären Amin ableitet oder ein Tetraalkylammoniumion bedeutet, das Kation $R^1$ gegen ein anderes austauscht.